# FASCICULE DE BREVET EUROPEEN

(11) **EP 1 667 986 B1**
(45) Date de publication et mention de la délivrance du brevet: **16.01.2013**
(21) Numéro de dépôt: 04787385.6
(22) Date de dépôt: 16.09.2004
(51) Int. Cl.: C07D 305/14, A61K 31/335

(54) **SOLVAT ACETONIQUE DU DIMETHOXY DOCETAXEL ET SON PROCEDE DE PREPARATION**
DIMETHOXYDOCETAXEL-ACETON-SOLVAT UND VERFAHREN ZU DESSEN HERSTELLUNG
DIMETHOXY DOCETAXEL ACETONE SOLVATE ET METHOD FOR THE PREPARATION THEREOF

(30) Priorité: 19.09.2003 FR 0311016
(43) Date de publication de la demande: 14.06.2006
(73) Titulaire: Aventis Pharma S.A., 92160 Antony (FR)
(72) Inventeur: DIDIER, Eric, F-75013 Paris (FR); PERRIN, Marc-Antoine, F-78350 Jouy en Josas (FR)
(74) Mandataire: Le Pennec, Magali
(86) Numéro de dépôt international: PCT/FR2004/002344
(87) Numéro de publication internationale: WO 2005/028462

(56) Documents cités:
- EP-A- 0 982 027
- WO-A-96/30355
- WO-A-97/32869

## Description

La présente invention concerne le solvat acétonique du diméthoxy docétaxel ou (2R,3S)-3-tert-butoxycarbonylamino-2-hydroxy-3-phénylpropionate de 4-acétoxy-2α-benzoyloxy-5β,20-époxy-1-hydroxy-7β,10β-diméthoxy-9-oxo-tax-11-ène-13α-yle, et son procédé de préparation.

Le document WO9732869 décrit le composé (2R,3S)-3-tert-butoxycarbonylamino-2-hydroxy-3-phénylpropionate de 4-acétoxy-2α-benzoyloxy-5β,20-époxy-1-hydroxy-7β,10α-diméthoxy-9-oxo-tax-11-ène-13α-yle. Ce dernier est un stéréoisomère du (2R,3S)-3-tert-butoxycarbonylamino-2-hydroxy-3-phénylpropionate de 4-acétoxy-2α-benzoyloxy-5β,20-époxy-1-hydroxy-7β,10β-diméthoxy-9-oxo-tax-11-ène-13α-yle.

Le document EP0982027 décrit l'utilisation du composé (2R,3S)-3-tert-butoxycarbonylamino-2-hydroxy-3-phénylpropionate de 4-acétoxy-2α-benzoyloxy-5β,20-époxy-1-hydroxy-7β,10β-diméthoxy-9-oxo-tax-11-éne-13α-yle pour le traitement de la prolifération anormale de cellules dans le cerveau.

Le (2R,3S)-3-tert-butoxycarbonylamino-2-hydroxy-3-phénylpropionate de 4-acétoxy-2α-benzoyloxy-5β,20-époxy-1-hydroxy-7β,10β-diméthoxy-9-oxo-tax-11-éne-13α-yle présente des propriétés anticancéreuses et antileucémiques remarquables.

Le (2R,3S)-3-tert-butoxycarbonylamino-2-hydroxy-3-phénylpropionate de 4-acétoxy-2α-benzoyloxy-5β,20-époxy-1-hydroxy-7β,10β-diméthoxy-9-oxo-tax-11-ène-13α-yle est préparé selon le procédé qui est décrit plus particulièrement dans la demande internationale PCT WO 96/30355 ou la demande internationale PCT WO 99/25704, selon le procédé décrit dans ces demandes le produit n'est pas cristallisé et n'est pas caractérisé.

Il a été trouvé que le solvat acétonique du (2R,3S)-3-ter-butoxycarbonylamino-2-hydroxy-3-phénylpropionate de 4-acétoxy-2α-benzoyloxy-5β,20-époxy-1-hydroxy-7β,10β-diméthoxy-9-oxo-tax-11-ène-13α-yle est parfaitement caractérisé d'un point de vue chimique.

Selon l'invention, le solvat acétonique du (2R,3S)-3-ter-butoxycarbonylamino-2-hydroxy-3-phénylpropionate de 4-acétoxy-2α-benzoyloxy-5β,20-époxy-1-hydroxy-7β,10β-diméthoxy-9-oxo-tax-11-ène-13α-yle peut être obtenu par cristallisation du (2R,3S)-3-tert-butoxycarbonylamino-2-hydroxy-3-phénylpropionate de 4-acétoxy-2α-benzoyloxy-5β,20-époxy-1-hydroxy-7β,10β-diméthoxy-9-oxo-tax-11-ène-13α-yle dans un mélange d'eau et d'acétone suivi du séchage sous pression réduite du produit isolé.

Pour la mise en oeuvre du procédé selon l'invention, il peut être particulièrement avantageux
- de mettre en solution le (2R,3S)-3-tert-butoxycarbonylamino-2-hydroxy-3-phénylpropionate de 4-acétoxy-2α-benzoyloxy-5β,20-époxy-1-hydroxy-7β,10β-diméthoxy-9-oxo-tax-11-ène-13α-yle dans l'acétone,
- de traiter la solution par de l'eau,
- d'ensemencer la solution avec une suspension dudit produit dans un mélange acétone/eau puis de traiter de nouveau à l'eau,
- de séparer les cristaux obtenus, puis
- de les sécher sous pression réduite.

Généralement, le (2R,3S)-3-tert-butoxycarbonylamino-2-hydroxy-3-phényl-propionate de 4-acétoxy-2α-benzoyloxy-5β,20-époxy-1-hydroxy-7β,10β-diméthoxy-9-oxo-tax-11-ène-13α-yle est dissous dans l'acétone. De préférence, la quantité d'acétone est comprise entre 5 et 20 parties en volume (ml) par rapport au poids (en gramme) de (2R,3S)-3-tert-butoxycarbonylamino-2-hydroxy-3-phénylpropionate de 4-acétoxy-2α-benzoyloxy-5β,20-époxy-1-hydroxy-7β,10β-diméthoxy-9-oxo-tax-11-ène-13α-yle mis en oeuvre (idéalement 10).

L'ensemencement préféré se fait à une concentration de 60 à 80 g (idéalement 68 g) par litre de mélange contenant un ratio volumique acétone/eau compris entre 65/35 à 75/25 et préférentiellement d'environ 68/32. Le mélange volumique acétone/eau en fin de précipitation est compris entre 70/30 minimum et 30/70 maximum (idéalement 45/55). L'ensemble du processus de cristallisation se déroule selon une meilleure manière de mettre en oeuvre l'invention à 20 ± 5°C (idéalement 20°C).

Le solvat acétonique du (2R,3S)-3-tert-butoxycarbonylamino-2-hydroxy-3-phényl-propionate de 4-acétoxy-2α-benzoyloxy-5β,20-époxy-1-hydroxy-7β,10β-diméthoxy-9-oxo-tax-11-ène-13α-yle qui cristallise est séparé, de préférence par filtration ou centrifugation. Le séchage est effectué sous pression réduite, comprise généralement entre 0,5 et 30 kPa, de préférence voisine de 0,7 kPa à une température comprise entre 30 et 60°C, de préférence voisine de 40°C.

Le séchage du produit a été étudié. Ainsi des échantillons de solvat acétonique du (2R,3S)-3-tert-butoxycarbonylamino-2-hydroxy-3-phénylpropionate de 4-acétoxy-2α-benzoyloxy-5β,20-époxy-1-hydroxy-7β,10β-diméthoxy-9-oxo-tax-11-ène-13α-yle volontairement traités à une température au dessus de 70°C (70 à 100°C) montre une perte grandissante de la teneur en acétone avec l'augmentation de la température. Pour le séchage ainsi une température préférée est comprise entre 30 et 60°C et encore plus préférentiellement est voisine de 40°C. Une valeur moyenne de la teneur en acétone est de 7 % ce qui représente à peu près la stoechiométrie en acétone qui est de 6.5 % pour un solvat à une molécule d'acétone.

La présente invention sera plus complètement décrite à l'aide des exemples suivants qui ne doivent pas être considérés comme limitatifs de l'invention.

### Exemple 1

A une solution de 207 g de (2R,3S)-3-tert-butoxycarbonylamino-2-hydroxy-3-phénylpropionate de 4-acétoxy-2α-benzoyloxy-5β,20-époxy-1-hydroxy-7β,10β-diméthoxy-9-oxo-tax-11-ène-13α-yle, à environ 92 % en poids dans environ 2 litres d'acétone, on ajoute à 20 ± 5°C température ambiante, 940 ml d'eau purifiée puis on ensemence avec une suspension de 2 g de (2R,3S)-3-tert-butoxycarbonylamino-2-hydroxy-3-phénylpropionate de 4-acétoxy-2α-benzoyloxy-5β,20-époxy-1-hydroxy-7β, 10β-diméthoxy-9-oxo-tax-11-ène-13α-yle isolé dans acétone/eau dans un mélange de 20 ml d'eau et 20 ml d'acétone. On laisse agiter environ 10 à 22 heures et on additionne en 4 à 5 heures 1,5 litres d'eau purifiée. On laisse agiter 60 à 90 minutes puis la suspension est filtrée sous pression réduite. Le gâteau est lavé sur filtre avec une solution préparée à partir de 450 ml d'acétone et 550 ml d'eau purifiée puis séché en étuve à 55°C sous pression réduite (0,7 kPa) pendant 4 heures. On obtient 197 g de (2R,3S)-3-tert-butoxycarbonylamino-2-hydroxy-3-phénylpropionate de 4-acétoxy-2α-benzoyloxy-5β,20-époxy-1-hydroxy-7β,10β-diméthoxy-9-oxo-tax-11-ène-13α-yle, acétone contenant 0,1 % d'eau et 7,2 % d'acétone (théorie de 6,5 % pour un solvat stoéchiométrique).

### Etude de séchage

Le produit est de nouveau mis en étuve et séché successivement, 18 heures à 60°C sous pression réduite de 0,7 kPa, 3 heures à 60°C sous une humidité relative d'environ 80 % (pression réduite de 160 mmHg), 18 heures à 70°C sous une humidité relative d'environ 80 % (pression réduite de 200 mmHg). A ce stade, la teneur en eau est de 0,2 % et la teneur en acétone de 4,7 % (194 g). A ce même stade, 1 aliquot de 1 g du lot est séché sous pression réduite de 5 mmHg successivement 18 heures à 80°C (teneur résiduelle en acétone de 0,5 %) puis 21 heures à 100°C (teneur résiduelle en acétone de 0,02 %). Le restant est séché à 80°C sous pression réduite de 5 mmHg pendant 31 heures (acétone 1,7 %, eau 0,3 %, titre sur tel de 96,5 %, pureté supérieure à 99 %).

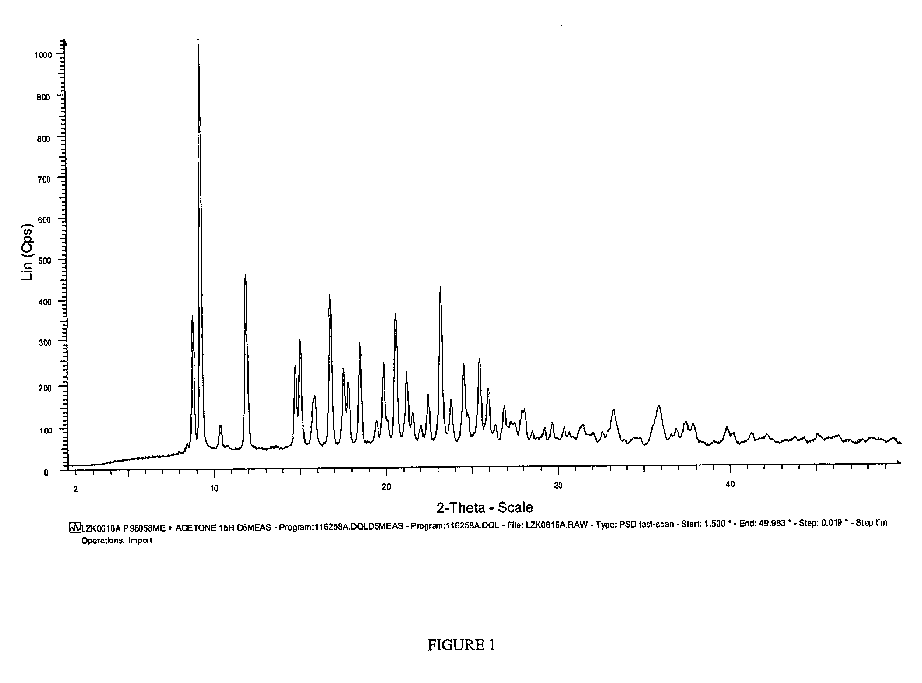

### Conditions opératoires utilisées pour l'acquisition du diagramme (figure 1) :

Les analyses sont effectuées sur le diffractomètre Bruker D5000 équipé d'une chambre en température Anton-Paar TTK. Le montage en réflexion est à géométrie focalisante de type Bragg-Brentano (*θ*-*θ*). La poudre est déposée sur un porte échantillon creux en aluminium. Un tube à anticathode de cobalt (40kV/30mA) fournit un rayonnement incident filtré par le fer. Deux radiations sont émises : Co *K*α₁, (λ, = 1,7890 Å) et Co *K*α₂ (λ = 1,7929 Å). Le filtrage par le fer n'élimine pas totalement la radiation *Kβ* (λ = 1,6208 Å pour le cobalt) qui participe encore au rayonnement incident à hauteur de 1 % (donnée constructeur) de l'intensité du doublet *K*α*.*

Des fentes de Soller améliorent le parallélisme du faisceau. Des fentes avant variables permettent de conserver une surface d'illumination constante de l'échantillon. Un collimateur de 1 mm limite la diffusion entre le tube et la chambre de mesure. Un détecteur linéaire Braun 50 M multicanal est utilisé. Il présente une fenêtre de détection d'une largeur de 10° en angle 2*θ*. Les conditions d'enregistrement des diagrammes sont les suivantes : balayage de 1,5 à 50 degrés en 2*θ*, temps de comptage de 30 secondes par degré en 2*θ*, dans les conditions ambiantes de température, pression et humidité relative.

La figure 1 représente le diagramme DRXP de référence de la forme solvate à l'acétone (forme A) du produit de l'exemple 1.

Spectre RMN du produit de l'exemple 1

¹H NMR Spectrum (400 MHz, CDCl₃, δ in ppm) : 1,20 (s : 3H) ; 1,22 (s : 3H) ; 1,37 (s : 9H) ; 1,67 (s : 1H); 1,72 (s : 3H) ; 1,80 (mt : 1H); 1,88 (s : 3H) ; 2,17 (s : 6H) ; de 2,20 à 2,40 (mt : 2H) ; 2,36 (s : 3H) ; 2,70 (mt : 1H) ; 3,30 (s : 3H) ; 3,46 (s : 3H) ; 3,47 (mt : 1H) ; 3,82 (d, J = 7,5 Hz : 1H) ; 3,86 (dd, J = 11 et 6,5 Hz : 1H) ; 4,17 (d, J = 8,5 Hz : 1H) ; 4,30 (d, J = 8,5 Hz : 1H) ; 4,63 (mt : 1H) ; 4,80 (s : 1H) ; 4,97 (d large, J = 10 Hz : 1H) ; 5,27 (d large, J = 10 Hz : 1H) ; 5,44 (d, J =10 Hz : 1H) ; 5,64 (d, J = 7,5 Hz : 1H) ; 6,21 (t, J = 9 Hz : 1H) ; de 7,25 à 7,45 (mt : 5H) ; 7,49 (t, J = 7,5 Hz : 2H) ; 7,60 (t large, J = 7,5 Hz : 1H) ; 8,09 (d, J = 7,5 Hz : 2H).

## Revendications

1. Solvat acétonique du (2R,3S)-3-ter-butoxycarbonylamino-2-hydroxy-3-phénylpropionate de 4-acétoxy-2α-benzoyloxy-5β,20-époxy-1-hydroxy-7β,10β-diméthoxy-9-oxo-tax-11-ène-13α-yle.

2. Solvat acétonique du (2R,3S)-3-ter-butoxycarbonylamino-2-hydroxy-3-phénylpropionate de 4-acétoxy-2α-benzoyloxy-5β,20-époxy-1-hydroxy-7β,10β-diméthoxy-9-oxo-tax-11-ène-13α-yle contenant entre 5 et 7% en poids d'acétone.

3. Procédé de préparation du solvat acétonique du (2R,3S)-3-tert-butoxycarbonylamino-2-hydroxy-3-phénylpropionate de 4-acétoxy-2α-benzoyloxy-5**,**20-époxy-1-hydroxy-7β,10β-diméthoxy-9-oxo-tax-11-ène-13α-yle **caractérisé en ce que** l'on cristallise le (2R,3S)-3-tert-butoxycarbonylamino-2-hydroxy-3-phénylpropionate de 4-acétoxy-2α-benzoyloxy-5β,20-époxy-1-hydroxy-7β,10β-diméthoxy-9-oxo-tax-11-ène-13α-yle dans un mélange d'eau et d'acétone, que on ensemence la solution avec une suspension dudit produit dans un mélange acétone/eau puis ensuite on traite à l'eau et que l'on sèche le produit obtenu sous pression réduite.

4. Procédé selon la revendication 3 caractérisé en que l'ensemencement se fait à une concentration de 60 à 80 g par litre d'un mélange contenant un ratio volumique acétone/eau compris entre 65/35 à 75/25.

5. Procédé selon la revendication 4 caractérisé en que l'ensemencement se fait dans un mélange contenant un ratio volumique acétone/eau d'environ 68/32.

6. Procédé selon la revendication 3 **caractérisé en ce que** le mélange volumique acétone/eau en fin de précipitation est compris entre 70/30 minimum et 30/70 maximum.

7. Procédé selon la revendication 6 **caractérisé en ce que** le mélange volumique acétone/eau en fin de précipitation est d'environ 45/55.

8. Procédé selon l'une quelconque des revendications 3 à 7 **caractérisé en ce que** le processus de cristallisation se déroule à 20 ± 5°C.

9. Procédé selon la revendication 2 **caractérisé en ce que** le séchage est effectué à une température comprise entre 30 et 60°C et encore plus préférentiellement est voisine de 40°C sous une pression voisine de 0,7 kPa.

10. Procédé selon la revendication 2 **caractérisé en ce que** l'on opère directement à partir de la solution acétonique du (2R,3S)-3-tert-butoxycarbonylamino-2-hydroxy-3-phénylpropionate de 4-acétoxy-2α-benzoyloxy-5β,20-époxy-1-hydroxy-7β,10β-diméthoxy-9-oxo-tax-11-ène-13α-yle obtenu par déprotection en milieu acide de l'ester (2R,4S,5R)-3-tert-butoxycarbonyl-2-(4-méthoxyphényl)-4-phényloxazolidine-5-carboxylate de 4-acétoxy-2α-benzoyloxy-5β,20-époxy-1-hydroxy-7β,10β-diméthoxy-9-oxo-tax-11-ène-13α-yle.

## Claims

1. Acetone solvate of 4-acetoxy-2α-benzoyloxy-5β,20-epoxy-1-hydroxy-7β,10β-dimethoxy-9-oxotax-11-en-13α-yl (2R,3S)-3-tert-butoxycarbonylamino-2-hydroxy-3-phenylpropionate.

2. Acetone solvate of 4-acetoxy-2α-benzoyloxy-5β,20-epoxy-1-hydroxy-7β,10β-dimethoxy-9-oxotax-11-en-13α-yl (2R,3S)-3-tert-butoxycarbonylamino-2-hydroxy-3-phenylpropionate comprising between 5 and 7% by weight of acetone.

3. Process for the preparation of the acetone solvate of 4-acetoxy-2α-benzoyloxy-5β,20-epoxy-1-hydroxy-7β,1 10β-dimethoxy-9-oxotax-11-en-13α-yl (2R,3S)-3-tert-butoxycarbonylamino-2-hydroxy-3-phenylpropionate, **characterized in that** 4-acetoxy-2α-benzoyloxy-5β,20-epoxy-1-hydroxy-7β,10β-dimethoxy-9-oxotax-11-en-13α-yl (2R,3S)-3-tert-butoxycarbonylamino-2-hydroxy-3-phenylpropionate is crystallized from a mixture of water and of acetone, **in that** the solution is seeded with a suspension of said product in an acetone/water mixture and then subsequently treatment is carried out with water, and **in that** the product obtained is dried under reduced pressure.

4. Process according to Claim 3, **characterized in that** the seeding is carried out at a concentration of 60 to 80 g per liter of a mixture comprising an acetone/water ratio by volume of between 65/35 to 75/25.

5. Process according to Claim 4, **characterized in that** the seeding is carried out in a mixture comprising an acetone/water ratio by volume of about approximately 68/32.

6. Process according to Claim 3, **characterized in that** the acetone/water mixture by volume at the end of precipitation is between 70/30 minimum and 30/70 maximum.

7. Process according to Claim 6, **characterized in that** the acetone/water mixture by volume at the end of precipitation is approximately 45/55.

8. Process according to any one of Claims 3 to 7, **characterized in that** the crystallization process takes place at 20 ± 5°C.

9. Process according to Claim 2, **characterized in that** drying is carried out at a temperature of between 30 and 60°C and more preferably still in the region of 40°C under a pressure in the region of 0.7 kPa.

10. Process according to Claim 2, **characterized in that** the preparation is carried out directly starting from the acetone solution of 4-acetoxy-2α-benzoyloxy-5β,20-epoxy-1-hydroxy-7β,10β-dimethoxy-9-oxotax-11-en-13α-yl (2R,3S)-3-tert-butoxycarbonylamino-2-hydroxy-3-phenylpropionate obtained by deprotection in an acid medium of the ester 4-acetoxy-2α-benzoyloxy-5β,20-epoxy-1-hydroxy-7β, 10β-dimethoxy-9-oxotax-11-en-13α-yl (2R,4S,SR)-3-tert-butoxycarbonyl-2-(4-methoxyphenyl)-4-phenyloxazolidine-5-carboxylate.

## Patentansprüche

1. Acetonsolvat von (2R,3S)-3-tert.-Butoxycarbonylamino-2-hydroxy-3-phenylpropionsäure-4-acetoxy-2α-benzoyloxy-5β,20-epoxy-1-hydroxy-7β,10β-dimethoxy-9-oxotax-11-en-13a-ylester.

2. Acetonsolvat von (2R,3S)-3-tert.-Butoxycarbonylamino-2-hydroxy-3-phenylpropionsäure-4-acetoxy-2α-benzoyloxy-5β,20-epoxy-1-hydroxy-7β,10β-dimethoxy-9-oxotax-11-en-13α-ylester, das zwischen 5 und 7 Gew.-% Aceton enthält.

3. Verfahren zur Herstellung des Acetonsolvats von (2R,3S)-3-tert.-Butoxycarbonylamino-2-hydroxy-3-phenylpropionsäure-4-acetoxy-2α-benzoyloxy-5β,20-epoxy-1-hydroxy-7β,10β-dimethoxy-9-oxotax-11-en-13α-ylester, **dadurch gekennzeichnet, dass** man (2R,3S)-3-tert.-Butoxycarbonylamino-2-hydroxy-3-phenylpropionsäure-4-acetoxy-2α-benzoyloxy-5β,20-epoxy-1-hydroxy-7β,10β-dimethoxy-9-oxotax-11-en-13α-ylester aus einer Mischung von Wasser und Aceton kristallisiert, die Lösung mit einer Susspension des Produkts in einer Aceton/Wasser-Mischung beimpft und anschließend mit Wasser behandelt und das erhaltene Produkt unter vermindertem Druck trocknet.

4. Verfahren nach Anspruch 3, **dadurch gekennzeichnet, dass** die Beimpfung bei einer Konzentration von 60 bis 80 g pro Liter einer Mischung, die ein Aceton/Wasser-Volumenverhältnis zwischen 65/35 und 72/25 enthält, erfolgt.

5. Verfahren nach Anspruch 4, **dadurch gekennzeichnet, dass** die Beimpfung in einer Mischung, die ein Aceton/Wasser-Volumenverhältnis von ungefähr 68/32 aufweist, erfolgt.

6. Verfahren nach Anspruch 3, **dadurch gekennzeichnet, dass** die Aceton/Wasser-Volumenmischung am Ende der Fällung zwischen minimal 70/30 und maximal 30/70 liegt.

7. Verfahren nach Anspruch 6, **dadurch gekennzeichnet, dass** die Aceton/Wasser-Volumenmischung am Ende der Fällung ungefähr 45/55 ist.

8. Verfahren nach einem der Ansprüche 3 bis 7, **dadurch gekennzeichnet, dass** der Kristallisationsprozess bei 20 ± 5°C stattfindet.

9. Verfahren nach Anspruch 2, **dadurch gekennzeichnet, dass** das Trocknen bei einer Temperatur zwischen 30 und 60°C und noch weiter bevorzugt in der Nähe von 40°C unter einem Druck in der Nähe von 0,7 kPa erfolgt.

10. Verfahren nach Anspruch 2, **dadurch gekennzeichnet, dass** man direkt von der durch Entschützung von (2R,4S,5R)-3-tert.-Butoxycarbonyl-2-(4-methoxyphenyl)-4-phenyloxazolidin-5-carbonsäure-4-acetoxy-2α-benzoyloxy-5β,20-epoxy-1-hydroxy-7β,10β-dimethoxy-9-oxotax-11-en-13α-ylester in saurem Medium erhaltenen Acetonlösung von (2R,3S)-3-tert.-Butoxycarbonylamino-2-hydroxy-3-phenyl-propionsäure-4-acetoxy-2α-benzoyloxy-5β,20-epoxy-1-hydroxy-7β,10β-dimethoxy-9-oxotax-11-en-13α-yl-ester ausgeht.
